# EUROPÄISCHE PATENTSCHRIFT

(11) **EP 0 481 103 B1**
(45) Veröffentlichungstag und Bekanntmachung des Hinweises auf die Patenterteilung: **21.12.1994**
(21) Anmeldenummer: 90119743.4
(22) Anmeldetag: 15.10.1990
(51) Int. Cl.: A61B 6/03, H01J 35/14

(54) **Röntgencomputertomograph mit ringförmig geführtem Elektronenstrahl**
X-ray computer tomograph with a circular path
Tomodensitomètre avec faisceau électronique dévié suivant une trajectoire circulaire

(43) Veröffentlichungstag der Anmeldung: 22.04.1992
(73) Patentinhaber: SIEMENS AKTIENGESELLSCHAFT, 80333 München (DE)
(72) Erfinder: Krumme, Hans-Jochen, Dipl.-Ing., W-8525 Uttenreuth (DE)

(56) Entgegenhaltungen:
- EP-A- 0 232 056
- GB-A- 2 044 985
- US-A- 3 919 550

## Beschreibung

Es ist bekannt, einen Computertomographen in der Weise auszubilden, daß eine ein Meßfeld umgebende ringförmige Röntgenstrahlenquelle vorgesehen wird, in der eine Ringanode angeordnet ist, die zur Erzeugung eines sich drehenden Röntgenstrahlenbündels von einem Elektronenstrahl abgetastet wird. Die Elektronenkanone zum Einschießen des Elektronenstrahles ist dabei auf der Meßfeldachse, die gleichzeitig die Achse der Ringanode bildet, im axialen Abstand von der Ringanode angeordnet, so daß sich insgesamt ein trichterförmiger Aufbau der Röntgenstrahlenquelle und damit eine relativ große Baulänge ergibt.

In der GB-A-2 044 985 ist ein Röntgencomputertomograph mit einer ein Meßfeld umgebenden ringförmigen Röntgenstrahlenquelle, in der eine Ringanode angeordnet ist, die zur Erzeugung eines sich drehenden Röntgenstrahlenbündels von einem Elektronenstrahl abgetastet wird, beschrieben, wobei der Elektronenstrahl auf einem Strahlfänger auftrifft. Der Elektronenstrahl wird dabei durch Ablenkmittel derart gesteuert, daß er entweder die Röntgenstrahlenquelle konzentrisch zur Ringanode durchsetzt, bis er auf dem Strahlfänger auftrifft, oder unter Fokussierung auf die Ringanode diese kontinuierlich abtastet. Der Elektronenstrahl erzeugt auf der Ringanode vom jeweiligen Brennfleck ausgehend ein fächerförmig eingeblendetes Röntgenstrahlenbündel, das sich um die Meßfeldachse dreht und das Untersuchungsobjekt unter verschiedenen Winkeln abtastet. Ein ringförmiger Röntgenstrahlendetektor erfaßt die aus dem Untersuchungsobjekt austretende Röntgenstrahlung und führt entsprechende Signale einem Rechner zu, der daraus ein Bild der untersuchten Schicht des Untersuchungsobjektes berechnet.

Der Elektronenstrahl kann typischerweise eine Stromstärke in der Größenordnung bis zu 1 A bei Energien von 100 bis 120 KeV haben. Um die für die Computertomographie notwendigen Brennflecke zu erhalten, kann der Querschnitt des Elektronenstrahles kreisförmig oder elliptisch mit Durchmessern von ca. 0,5 bis 1 mm sein. Der Computertomograph benötigt einen Röntgenstrahl, der um den Patienten mit einer Scanzeit von 30 bis 100 ms rotiert, wobei in dieser Zeit mindestens ein Winkel von 180 plus Fächerwinkel, also beispielsweise 220°, umschrieben werden muß.

Um den Elektronenstrahl ringförmig zu führen, müssen entsprechende Ablenkmittel (z.B. Spulen) vorgesehen werden. Trotzdem tendiert der Elektronenstrahl infolge Raumladung zu einer zusätzlichen Strahlaufspreizung, die durch die als Fokussiereinrichtung wirkenden Ablenkmittel nicht völlig ausgeglichen werden kann.

Die Raumladung der Elektronen kann durch Ionen kompensiert oder neutralisiert werden. Dies geschieht beispielsweise durch Einleitung von Gasen in das Vakuumgefäß der Röntgenstrahlenquelle, wobei Drücke von größenordnungsmäßig 10⁻⁵ bis 10⁻⁶ Millibar angewendet werden. Die Gase werden durch Interaktion mit dem Elektronenstrahl ionisiert. Diese so entstehenden sogenannten Restgasionen werden in einer Zeit von einigen µs nach Einschalten des Elektronenstrahles erzeugt und sorgen dann für eine Selbstfokussierung des Elektronenstrahles.

Würde man den Elektronenstrahl im Vorlauf für die Röntgenstrahlerzeugung nutzen, könnte sich dort keine Ionisierung aufbauen. Damit würde dort auch keine Selbstfokussierung stattfinden. Der Elektronenstrahl würde divergieren und nach einigen Zentimetern die Gehäusewände treffen.

Die Fokussierungsanforderungen sind nur dann weniger kritisch, wenn der Elektronenstrahl im Ringkanal mit einem größeren Durchmesser als 1 mm, also z.B. einigen Millimetern, geführt wird. Durch geeignete zusätzliche magnetische Felder muß dann eine Anpassung der Fokussierung beim Einschuß der Elektronen in die Elektronenbahn und beim Auslenken auf die Ringanode vorgenommen werden. Es ist in diesem Fall aber nicht leicht, eine den Anforderungen entsprechende, über die gesamte Ringanode konstante Brennfleckgröße zu erzeugen.

Der Erfindung liegt die Aufgabe zugrunde, einen Röntgencomputertomographen der eingangs genannten Art so auszubilden, daß eine gut fokussierte Abtastung der Ringanode erfolgt.

Diese Aufgabe ist erfindungsgemäß gelöst durch die Merkmale des Patentanspruches 1. Dabei werden vor der Abtastung der Ringanode über deren gesamte Länge Ionen gebildet, die dann ihre Aufgabe zur Fokussierung und Stabilisierung des Elektronenstrahles erfüllen können. Röntgenstrahlung entsteht zunächst noch nicht. Der Aufbau des Elektronenstrahles dauert einige µs. Nach 10 bis 20 µs wird der Elektronenstrahl durch Einschalten eines entsprechend wandernden Magnetfeldes von der dem Strahlfänger zugewandten Seite der Ringanode beginnend auf diese stetig abgelenkt, wo er durch seinen Aufprall Röntgenstrahlung erzeugt. Das Röntgenstrahlenbündel und dessen Fokus wandern von dem dem Strahlfänger zugewandten Ende der Röntgenstrahlenquelle aus kontinuierlich bis zu deren anderem Ende.

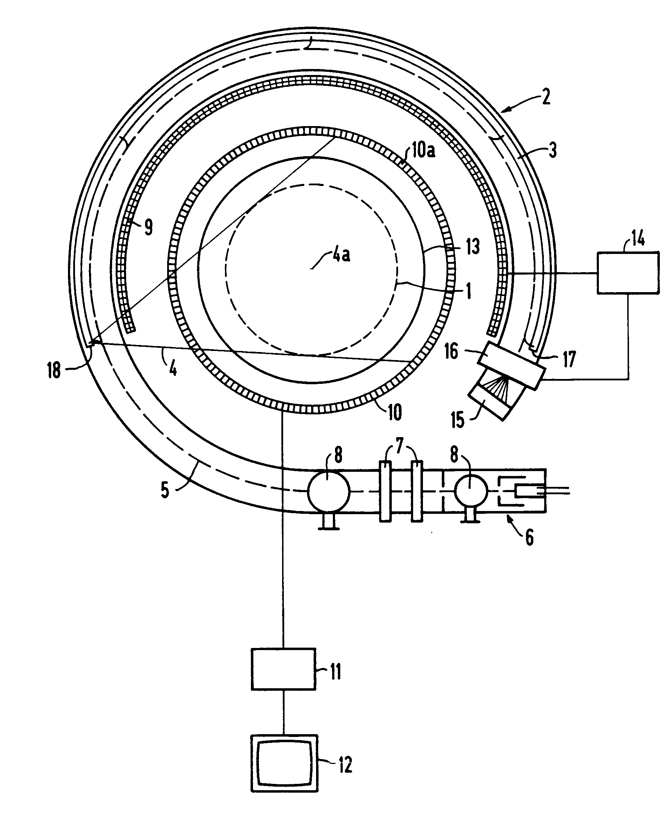

Die Erfindung ist nachfolgend anhand eines in der Zeichnung dargestellten Ausführungsbeispieles näher erläutert.

In der Zeichnung ist ein Röntgencomputertomograph mit einer ein Meßfeld 1 umgebenden, ringförmigen Röntgenstrahlenquelle 2 dargestellt, in der eine Ringanode 3 angeordnet ist. Die Ringanode 3 wird zur Erzeugung eines sich drehenden, fächerförmigen Röntgenstrahlenbündels 4 von einem Elektronenstrahl 5 abgetastet, der von einer Elektronenkanone 6 erzeugt wird. Der Elektronenkanone 6 sind Fokussierungsspulen 7 nachgeschaltet. Das Vakuum in der Röntgenstrahlenquelle 2 wird durch Vakuumpumpen 8 aufrecht erhalten. Der Elektronenstrahl 5 wird zur Erzeugung des Röntgenstrahlenbündels 4 mit Hilfe einer magnetischen Ablenkspule 9 auf die Ringanode 3 abgelenkt. Die aus dem Untersuchungsobjekt im Meßfeld 1 austretende Röntgenstrahlung wird von einem ringförmigen Strahlendetektor 10, der aus einer Reihe von Detektorelementen besteht, erfaßt. Die Ausgangssignale der Detektorelemente 10a usw. werden einem Rechner 11 zugeführt, der daraus ein Bild der untersuchten Schicht des Untersuchungsobjektes berechnet und auf einem Monitor 12 wiedergibt. Das Meßfeld 1 ist ein Feld in einer Öffnung 13, in die das Untersuchungsobjekt eingeschoben wird. Das Röntgenstrahlenbündel 4 rotiert durch Ablenkung des Elektronenstrahles 5 auf die Ringanode 3 zur Durchstrahlung des Untersuchungsobjektes unter verschiedenen Richtungen um die Achse 4a.

Eine Steuervorrichtung 14 steuert die Ablenkspule 9 in der Weise, daß der Elektronenstrahl 5 vor Beginn eines Abtastvorganges die Röntgenstrahlenquelle 2 konzentrisch zur Ringanode 3 durchsetzt, bis er am geschlossenen Ende auf einem Strahlenfänger 15, z.B. aus Blei, auftrifft. Vorher wird er durch eine Defokussiereinrichtung 16 defokussiert. Anschließend wird er durch die Ablenkspule 9 auf die Ringanode 3 abgelenkt und tastet diese von ihrem Ende 17 zu ihrem Ende 18 ab. Fünf Fokuspositionen sind in der Zeichnung dargestellt. Tatsächlich sind es wesentlich mehr, z.B. 1 000. Das Röntgenstrahlenbündel 4 dreht sich also entgegen der Richtung des Elektronenstrahles 5 und befindet sich in der Zeichnung in seiner Endstellung. Hier ist ein Abtastvorgang beendet.

Danach erfolgt ein erneuter Aufbau des ringförmig geführten Elektronenstrahles 5, der wieder zunächst auf dem Strahlfänger 14 auftrifft. Mit der Ablenkung des Elektronenstrahles 5 auf das Ende 17 der Ringanode 3 beginnt ein neuer Abtastvorgang.

Die geschilderte Abtastung der Ringanode 3 sorgt für eine kontrollierte, stetige und stabile Fokussierung des Elektronenstrahles 5 über die gesamte Länge der Ringanode 3. Nur die letzte Weglänge des Elektronenstrahles 5 nach dem Abbiegen aus der ringförmigen Kreisbahn auf die Ringanode 3 findet keine Raumladungskompensation vor. Hier ist aber eine geringe Strahlaufspreizung (Divergenz) akzeptierbar, die ausgenutzt wird, um die gewünschte Fokusgröße auf der Ringanode 3 zu erreichen. Diese Fokusgröße ist dabei durch die elektrischen und geometrischen Werte der Röntgenstrahlenquelle festgelegt. Die Fokusgröße ist so über die gesamte Ringanode 3 von ihrem Ende 17 zu ihrem Ende 18 konstant.

Der Strahlendetektor 4 ist hinsichtlich der Ringanode 3 so angeordnet, daß das Röntgenstrahlenbündel an ihm vorbei passieren kann, bevor es in das Meßfeld 1 eintritt und erst nach seinem Austritt aus dem Meßfeld 1 auf dem Strahlendetektor 10 auftrifft.

## Patentansprüche

1. Röntgencomputertomograph mit einer ein Meßfeld (1) umgebenden ringförmigen Röntgenstrahlenquelle (2), in der eine Ringanode (3) angeordnet ist, die zur Erzeugung eines sich drehenden Röntgenstrahlenbündels (4) von einem Elektronenstrahl (5) abgetastet wird, wobei Ablenkmittel (9) für den Elektronenstrahl vorhanden sind, welcher auf einem Strahlfänger (14) auftrifft, **dadurch gekennzeichnet,** daß das Vakuumgefäß der Röntgenstrahlenquelle (2) ionisierbare Gase enthält, daß der Elektronenstrahl (5) derart gesteuert ist, daß er vor Beginn eines Abtastvorganges die Röntgenstrahlenquelle (2) konzentrisch zur Ringanode (3) durchsetzt, bis er am geschlossenen Ende auf dem Strahlfänger (14) auftrifft und anschließend von diesem Ende aus unter Fokussierung auf die Ringanode (3) diese abtastet, und daß die Verweildauer des Elektronenstrahles (5) auf dem Strahlfänger (14) derart bemessen ist, daß sich eine Ionisierung aufbauen kann.

2. Röntgencomputertomograph nach Anspruch 1, bei dem vor dem Strahlfänger (14) Mittel (16) zur Defokussierung des Elektronenstrahles (5) angeordnet sind.

3. Röntgencomputertomograph nach Anspruch 1 oder 2, bei dem die elektrischen und geometrischen Werte der Röntgenstrahlenquelle (2) so festgelegt sind, daß die Divergenz des Elektronenstrahles (5) nach dem Abbiegen aus der ringförmigen Kreisbahn die gewünschte Fokusgröße ergibt.

## Claims

1. An X-ray computer tomograph with an annular X-ray source 2, which surrounds a measuring field 1 and in which is arranged an annular anode (3) which is scanned by an electron beam (5) for the generation of a rotating X-ray beam (4), where deflecting beams (9) are provided for the electron beam which impinges upon a beam catcher (14), characterised in that the vacuum vessel of the X-ray source (2) contains ionizable gases, that the electron beam (5) is controlled in such manner that prior to the start of a scanning process it passes through the X-ray source (2) concentrically to the annular anode (3) until at the closed end it impinges upon the beam catcher (14) and then, from this end, being focused upon the annular anode (3), scans the annular anode (3), and that the dwell time of the electron beam (5) on the beam catcher (14) is contrived such that an ionization can build up.

2. An X-ray computer tomograph as claimed in Claim 1, wherein means (16) for defocusing the electron beam (5) are arranged preceding the beam catcher (14).

3. An X-ray computer tomograph as claimed in Claim 1 or 2, wherein the electrical and geometrical values of the X-ray source (2) are so defined that the divergency of the electron beam (5), having been bent out of the annular orbit, results in the desired focus size.

## Revendications

1. Tomodensitomètre comportant une source annulaire (2) de rayons X, qui entoure un champ de mesure (1) dans laquelle est disposée une anode annulaire (3), qui, pour la production d'un faisceau de rayons X tournant (4), est balayée par un faisceau d'électrons (5), il est prévu des moyens de déviation (9) du faisceau d'électrons, qui rencontre un récepteur de rayonnement (14), caractérisé par le fait que l'enceinte à vide de la source de rayons X (2) contient des gaz ionisables, que le faisceau d'électrons (5) est commandé de telle sorte qu'avant le début de l'opération de balayage, il traverse la source de rayons X (2) concentriquement à l'anode annulaire (3), avant de rencontrer, à l'extrémité fermée, le récepteur de rayonnement (14), et ensuite, à partir de cette extrémité, balaye l'anode annulaire (3) en étant focalisé sur cette dernière, et que la durée de séjour du faisceau d'électrons (5) sur le récepteur de rayonnement (14) est telle qu'il puisse se produire une ionisation.

2. Tomodensitomètre suivant la revendication 1, dans lequel des moyens (16) pour défocaliser le faisceau d'électrons (5) sont disposés en avant du récepteur de faisceau (14).

3. Tomodensitomètre suivant la revendication 1 ou 2, dans lequel les valeurs électriques et géométriques de la source de rayons X (2) sont fixées de telle sorte que la divergence du faisceau d'électrons (5) après déviation à partir de la trajectoire annulaire circulaire donne la taille de foyer souhaitée.
